# EUROPÄISCHE PATENTSCHRIFT

(11) **EP 0 934 998 B1**
(45) Veröffentlichungstag und Bekanntmachung des Hinweises auf die Patenterteilung: **02.11.2005**
(21) Anmeldenummer: 99101933.2
(22) Anmeldetag: 29.01.1999
(51) Int. Cl.: C12M 1/107, B09B 3/00

(54) **Verfahren und Vorrichtung zur Methanisierung von Biomassen**
Method and device for the methanation of biomasses
Procédé et dispositif pour la méthanisation de biomasses

(30) Priorität: 09.02.1998 DE 19805045
(43) Veröffentlichungstag der Anmeldung: 11.08.1999
(73) Patentinhaber: Hoffmann, Manfred, Prof. Dr., 91746 Weidenbach (DE); Institut für Agrartechnik Bornim e.V. ATB, 14469 Potsdam-Bornim (DE)
(72) Erfinder: Hoffmann, Manfred, Prof. Dr., 91746 Weidenbach (DE); Zürl, Berthold, 96184 Rentweinsdorf (DE); Linke, Bernd, Dr.sc.agr., Dipl.-Ing., 14469 Potsdam (DE); Schelle, Hannelore, Dipl.-Biochem., 14469 Potsdam (DE)
(74) Vertreter: Grättinger & Partner (GbR)

(56) Entgegenhaltungen:
- WO-A-89/08616
- WO-A-92/18261
- WO-A-96/12789
- DE-A- 19 532 359
- GB-A- 2 230 004
- US-A- 4 579 654

## Beschreibung

Die Erfindung bezieht sich auf ein Verfahren zur Verarbeitung von schüttfähiger, stapelbarer Biomasse, sowie eine Vorrichtung zur Durchführung dieses Verfahrens.

Die vorherrschende Technik bei der Methanisierung von Biomassen besteht darin, daß diese zunächst mit Preßwasser in einen pumpfähigen Zustand versetzt werden und dann in diesem pumpfähigen Zustand mit dem notwendigen Impfmaterial versetzt, durch Rührvorrichtungen ständig homogenisiert und zur Umsetzung umgepumpt werden. In diesem Sinne beschreibt beispielsweise die WO 96/12789 A1 ein Verfahren zur Vergärung von Biomasse, bei dem die zu methanisierende Biomasse während des Gärprozesses wiederkehrend umgewälzt wird. Demgemäß setzt das dieser Druckschrift entnehmbare Verfahren eine entsprechende Konditionierung (Zerkleinerung) der Biomasse voraus, um diese in einen pumpfähigen Zustand zu bringen. Auch das der US 4579654 A entnehmbare Verfahren zur Vergärung von Biomasse setzt voraus, daß diese pumpfähig, d.h. flüssig oder zumindest pastös ist. Solche Verfahren sind apparativ aufwendig und demzufolge auch teuer. Insbesondere eignen sie sich nicht oder nur bedingt für schüttfähige und stapelbare Biomassen und vor allem nicht für kleinräumige Anlagen zur direkten Methanisierung von Biomassen z.B. aus der Landschaftspflege, Straßenwartung, Bioabfallentsorgung und Festmistverwertung in der Landwirtschaft.

Die fließfähigen pumpfähigen Substrate (vorwiegend Gülle und kommunale Abfallstoffe) erfordern darüber hinaus auch eine externe Bereitstellung der Prozeßenergie für die Methanisierung.

Aus der Offenlegungsschrift DE 19532359 A1 ist ein Batch-Verfahren für die Biogaserzeugung aus feststoffreicher Biomasse, bei welchem über einen gemeinsamen Sammelkanal die Sickersäfte der einzelnen Fermenter einem Säurepuffer durch Umpumpen zugeführt werden, bekanntgeworden. Bei diesem Verfahren erfolgt auch eine Einstellung des Mischgehaltes an Nährstoffen und Methanbildnern. Diese Flüssigkeit wird dann über Pumpen weitergeleitet und als Perkolationsmedium wieder gezielt den Batch-Behältern zudosiert. Dabei kann das Perkolationsmedium ggf. auch temperiert angeboten werden.

Dieses vorbekannte Verfahren benötigt zwar keine Umsetzung der Biomasse in einen fließfähigen, d.h. pumpfähigen Zustand. Es bedarf aber nach wie vor eines erheblichen apparativen Aufwandes und einer während der gesamten Umsetzdauer anhaltenden Steuerung und Regelung der Pumpen zum Wiederzuführen der Sickersäfte als Perkolationsmedium zur Biomasse.

Aus der Offenlegungsschrift DE 4423099 A1 ist darüber hinaus ein Methanisierungsverfahren bekanntgeworden, bei dem eine laufende dosierte Zumischung von vorgewärmtem Preßwasser und/oder Restschlamm als Impfgut in das sich jeweils im Fermenter befindliche zugepumpte Substrat stattfindet. Dieses Impfgut wird im Kreislaufprozeß ständig im Wärmetauscher temperiert und rezirkulierend eingesetzt. Auch dieses Verfahren ist apparativ aufwendig und eignet sich nicht für die direkte Methanisierung von in kleineren Mengen anfallenden Biomassen direkt am Ort des Anfalls (z.B. Bauernhof, Lagerplatz).

Die WO 92/18261 beschreibt ein Verfahren zur Gewinnung von Biogas aus Biomasse. Diese wird hierzu in einzelne Biomasse-Ballen abgepackt, welche in einer spezifischen Schichtung in eine Deponie eingebaut werden. Eine stoffliche Nutzung des ausgegorenen Gärrückstands scheidet nach der Lehre dieses Dokuments aus.

Schließlich ist - man vergleiche hierzu beispielsweise die Zeitschrift "Müll und Abfall" 6/94 Seite 3 ff. - auch bereits ein als 3A-Verfahren bezeichnetes Verfahren zur Methanisierung von Biomassen vorgeschlagen worden. Dabei handelt es sich um ein Trockenfermentationsverfahren nach dem Schichten-Mietenverfahren, in welchem in drei Phasen (aerob = Kompostierung, anaerob = Vergärung, aerob = Kompostierung) jeweils in der aeroben Schicht eine Erwärmung und in der anaeroben Schicht eine Methanisierung rollierend stattfindet. Zwischen den einzelnen Schichten liegt fakultativ eine Trennschicht. Das Biogas wird abgesaugt. Eine Perkolationswasser-Kreuzlaufführung sorgt für die nötige Impfung. Auch dieses Verfahren eignet sich nur für die Methanisierung großer Massen an einer zentralen Verarbeitungsstelle. Es gibt hohe Methanverluste und es ist eine komplexe Umsetztechnologie erforderlich.

Der Erfindung liegt daher die Aufgabe zugrunde, ein besonders wirtschaftliches Verfahren zur Verarbeitung von schüttfähigen, stapelbaren oder stückigmachbaren Biomassen sowie eine zur Durchführung des Verfahrens geeignete Vorrichtung zu schaffen, bei der mit minimalem apparativen Aufwand und bei einfachster Verfahrensführung ohne Pumpen des Substrats eine Methanisierung auch in kleinen Chargen durchgeführt werden kann, so daß beispielsweise eine direkte Methanisierung von Biomassen in Preßballen, Flachlagern (Fahrsilo, Miststätte), Containern oder Hochsilos am Ort des Anfallens (z.B. direkt auf dem Bauernhof) und eine anschließende stoffliche Verwertung des Gärrückstands möglich ist.

Zur Lösung dieser Aufgabe umfaßt ein Verfahren zur Verarbeitung schüttfähiger, stapelbarer Biomasse
- eine aerobe Erwärmung der in einem Behälter angeordneten, von einer luftundurchlässigen Hülle umgebenen Biomasse,
- eine sich hieran anschließende Methanisierung der inoculierten, in dem Behälter angeordneten und von der luftundurchlässigen Hülle umgebenen Biomasse durch Vergären unter Luftabschluß nach Zusetzen eines lmpfmaterials als Einmalzugabe ohne weiteres Nachimpfen,
- Ausbringen des Restmaterials auf Freiflächen, ggfs. nach vorheriger Kompostierung und/oder Zwischenlagerung.

Demnach wird erfindungsgemäß die in einem Behälter (Ballen, Flachlager, Folien-Preßballen, Silo oder Abroll-Container) angeordnete, von einer luftundurchlässigen Hülle umgebene Biomasse sowohl aerob erwärmt als auch, anschließend hieran, methanisiert, wobei die Methanisierung der inoculierten Biomasse durch Vergären unter Luftabschluß nach Zusetzen eines Impfmaterials als Einmalzugabe ohne weiteres Nachimpfen erfolgt. Das Restmaterial wird, ggfs. nach vorheriger Kompostierung und/oder Zwischenlagerung, auf Freiflächen aufgebracht. Die bei diesem Verfahren erfolgende aerobe Erwärmung und anschließende Methanisierung der schüttfähigen, stapelbaren Biomasse wird nach einem weiteren Aspekt der vorliegenden Erfindung bevorzugt in einer Vorrichtung durchgeführt, die eine die Biomasse verpackende luftundurchlässige oder luftdurchlässige Hülle und eine dieses umgebende, als zweite, zumindest zeitweise gasdichte Hülle ausgebildete Wandung umfaßt, wobei sich zwischen der Transporthülle und der zweiten Hülle ein stehendes Gaspolster ausbilden kann.

Das erfindungsgemäße Verfahren, bei dem es weder darauf ankommt, daß große Mengen an Biomasse verfügbar sind, noch umständliche und teure Pumpsysteme zum Umpumpen der Reaktionsmassen notwendig sind, läßt sich dabei in unterschiedlicher Weise ausbilden.

Als Impfmaterial für das erstmalige Inbetriebnehmen des Verfahrens kann dabei vorteilhafterweise der Ablauf und/oder das Faulgut einer Biogasanlage verwendet werden, beispielsweise einer Biogasanlage, die Rindergülle verarbeitet, wobei mit besonderem Vorteil dem Impfmaterial strukturreiche Reststoffe beigemischt sein können, wie z.B. Grasschnitt, Strauchschnitt oder Stroh. Bei dieser Zumischung hat es sich als zweckmäßig erwiesen, daß die strukturreichen Reststoffe mit dem Ablauf aus einer landwirtschaftlichen Biogasanlage oder ausgefaultem Klärschlamm so vermischt werden, daß der Trockenmassegehalt im Gemisch 20 bis 40 Masse-%, vorzugsweise 30 %, beträgt und der pH-Wert zwischen 7 und 9, vorzugsweise bei 8, liegt. Durch die Einhaltung dieser genannten Verhältnisse wird ein Übersäuern der Reaktionsmasse im Verlauf der Methanisierung sicher verhindert. Ganz besonders vorteilhaft gestaltet sich mit derartigen schüttfähigen Impfmaterialien die nachfolgende, gesteuert auch zu einem späteren Zeitpunkt einleitbare Methanisierung der Reaktionsmasse, wenn das Verhältnis der Trockenmassen des Impfmaterials und der Biomasse größer als 0,6 ist.

Dabei können dem Gemisch stapelbare Biomassen, vorzugsweise organische Reststoffe aus der Lebensmittelindustrie oder Abfälle aus der Biotonne, in einer solchen Menge zugesetzt werden, daß noch ein Kompostierungsprozeß in Gang gesetzt werden kann und als Folge die Temperatur des Gemisches auf 40 bis 60°C, vorzugsweise 50°C, innerhalb von zwei bis fünf Tagen ohne Umsetzen ansteigt. Ganz allgemein gilt, daß das Impfmaterial in Abhängigkeit von der jeweiligen Biomasse hergestellt und zugesetzt werden sollte.

Als Impfmaterial für weitere Batch-Ansätze wird bevorzugt das Faulgut nach dem Ende der Methanisierung gemäß dem erfindungsgemäßen Verfahren verwendet und kann gemäß einer ersten Ausführungsform der vorliegenden Erfindung bereits vor dem Befüllen des Behälters, also insbesondere bereits bei der Herstellung von Preßballen oder eines Ladeschwads, mit der Biomasse vermischt werden, wobei die Bedingungen so gewählt werden, daß eine spontane Methanisierung ausgeschlossen ist. Dies wird entweder durch Luftzufuhr zu der im Behälter (Ballen) angeordneten Biomasse oder durch anaerobe Silierung erreicht. Bei entsprechender Vortrocknung und Lagerung bei Temperaturen unter 20°C kann man darüber hinaus auch verhindern, daß eine spontane Kernerwärmung z. B. des Preßballens stattfindet, so daß eine gezielte spätere Einleitung der Methanisierung sehr einfach durch entsprechende Temperatur- und ggf. Feuchtigkeitszufuhr möglich ist.

Bei Biomassen mit hohem Flüssigkeitsbindungsvermögen kann man auch ein fließfähiges Impfmaterial verwenden, wobei z.B. das Impfmaterial zwischen die Schichtpakete der Biomasse beim Pressen von Rund- oder Quaderballen eingebracht, insbesondere eingesprüht oder dem Ladeschwad zudosiert werden kann.

Darüber hinaus kann das fließfähige Impfmaterial auch in Form sich selbstauflösender "Nester" (z.B. Kunststoffbeutel) in die Biomasse eingebettet sein, wobei das Impfmaterial von einer sich allmählich auflösenden, beispielsweise bei entsprechender Temperatur sich zersetzenden Hülle umgeben sein kann. Durch die gezielte Wärmezufuhr kann die Auflösung der "Nester" eingeleitet und damit der Methanisierungsprozeß in Gang gesetzt werden.

Neben der Beimischung des Impfmaterials zur Biomasse bereits bei der Herstellung stückiger (Ballen) oder loser (Ladewagengut) Einheiten kann das Verfahren gemäß einer zweiten Ausführungsform der Erfindung auch in der Weise ausgestaltet sein, daß ein fließfähiges Impfmaterial verwendet wird, das der Biomasse nach Abschluß z. B. einer aeroben Kernerwärmung unter Verdrängung der in der Biomasse eingeschlossenen Luft derart zugeführt wird, daß die Temperatur der Reaktionsmasse einen Wert von ca. 40°C nicht unterschreitet und daß anschließend sofort die anaerobe Vergärungsstufe angeschlossen wird. Um die angesprochene Unterschreitung der Temperatur der Reaktionsmasse von etwa 40°C sicher zu verhindern, kann beispielsweise das Impfmaterial z.B.solar vorgewärmt sein. Es kann auch Prozeßabwärme von der Verbrennung des Biogases zugeführt werden.

Um die bei der Methanisierung gewünschte Temperatur von 35 - 37°C nicht zu unterschreiten, kann die Reaktionsmasse entweder nach außen thermisch isoliert sein oder aber vorgesehen sein, daß in der Methanisierungsstufe eine zusätzliche Erwärmung der Reaktionsmasse erfolgt. Dies kann beispielsweise dadurch geschehen, daß die Ballen mit einer kollektorartigen Außenhülle umgeben von der Sonne erwärmt werden, oder aber auch daß durch Sonnenkollektoren und/oder Warmwasserleitungen (Wärmeauskoppelung von Blockheizkraftwerken) die Behälteraußenwände, beispielsweise ein durch eine Folie abgedecktes Flachlager, erwärmt werden.

In weiterer Ausgestaltung der Erfindung kann vorgesehen sein, daß die anfallende Abtropfflüssigkeit (Perkolat) zur Temperatur-Homogenisierung und/oder Beimpfung und/oder zur Regulierung des Trockenmassengehaltes des Substrates zur Erreichung der Streufähigkeit für Stalldungstreuer der Reaktionsmasse rezirkulierend von oben zugefügt wird. Dabei hat es sich als besonders zweckmäßig erwiesen, wenn ein Teil des Perkolats in einem Perkolat-Pufferspeicher zwischengespeichert wird, um je nach Bedarf und unabhängig von dem Ausmaß des Anfalls des Perkolats von oben wieder über die Biomasse verteilt zu werden und wenn das Perkolat über Wärmetauscher temperiert wird.

Eine weitere Verbesserung des erfindungsgemäßen Verfahrens läßt sich dadurch erreichen, daß, vorzugsweise über die Begasungseinrichtung oder die Perkolatrezirkulation, spezielle Futterstoffe zur Kultivierung der Bakterienstämme dosiert zugesetzt werden. In Ergänzung dazu können, wiederum vorzugsweise über die Perkolatrezirkulation, Cofermente dosiert zugeführt werden.

Schließlich liegt es auch noch im Rahmen des erfindungsgemäßen Verfahrens unterschiedliche Biomassen, die jeweils für die Füllung eines eigenen Behälters nicht ausreichen, gemeinsam zu verarbeiten und dabei die Biomassen, beispielsweise Grasschnitt und Stroh einerseits und beispielsweise Hühnermist andererseits, durch Schichtung, kegelförmige Schüttung, keilförmiges oder röhrenförmiges Einbringen des Impfgutes so zu "mischen", daß eine optimale Methanisierung aller Komponenten stattfindet. Es wird also nicht eine homogene Vermischung der Biomassen vorgenommen, was häufig auch an der apparativen Ausstattung vor Ort scheitert, sondern es erfolgt die beschriebene geeignete Anordnung, die natürlich wiederum davon abhängt, welche unterschiedlichen Biomassen gleichzeitig miteinander verarbeitet werden sollen.

Zur Durchführung des erfindungsgemäßen Verfahrens soll die bei der Verarbeitung in Form von Preßballen in einer luftdurchlässigen (Garn- oder Netzbindung) Transportform oder einer luftdichten Wickelfolie verpackte Reaktionsmasse von einer zumindest zeitweise als gasdichte Hülle ausgebildeten Wandung umgeben sein, in der sich ein stehendes Gaspolster ausbildet. Dieses stehende Gaspolster isoliert die Reaktionsmasse nach außen, so daß die notwendige Temperatur aufrechterhalten bleibt, die für die anschließende Methanisierung erforderlich ist.

In einer anderen Ausführungsform einer Einrichtung zur Durchführung des erfindungsgemäßen Verfahrens kann schüttfähiges Substrat, z. B. Biomüll, in einem wasserdurchlässigen Sack (z. B. Trevira-Hängesack) so in einem Behälter einhängbar sein, dass er allseitig vom temperierten, flüssigen Impfmaterial umgeben ist, welches in das Substrat diffundiert.

Als besonders zweckmäßig hat sich dabei eine Vorrichtung erwiesen, bei der alle erforderlichen Perkolat-, Impfmaterial- und Gasführungen durch einen oder mehrere zentral oder radial angeordnete Einstechdorn(e) bzw. eine oder mehrere Zentralspule(n) mit oder ohne Flanschverbindungen erfolgen. Bei formbeständigen Biomassen kann dies auch direkt über Bohrungen erfolgen, die entweder durch anschließend wieder herausgezogene Einstechdorne oder sonstige geeignete Bohrer formstabil hergestellt werden. Schließlich ist es auch möglich, die Ver- und Entsorgungsbahnen im Inneren der bevorzugt als Preßballen ausgebildeten Reaktionsmassenansätze durch sich selbstauflösende Füllkörper zu bilden.

Die Ver- und Entsorgungsleitungen mehrerer Fermentereinheiten können mittels Schnellverschlüssen miteinander oder mit den zentralen Speichern bzw. Verdichtern gekoppelt werden, wobei ggf. Über- und/oder Unterdruckventile und Kondenswasserabscheider vorgesehen sein können. Neben dem einfachen Betrieb eines Behälters, beispielsweise eines Preßballens, können auch mehrere Behälter über spezielle Versorgungsköpfe gekoppelt sein, wobei die Kopplung bevorzugt so erfolgen kann, daß die mehreren Behälter gleichzeitig oder nacheinander in Serien- oder Parallelschaltung betrieben werden können.

Dabei liegt es auch noch im Rahmen der Erfindung, die Ver- und Entsorgungseinheiten in eine gemeinsame mehrschichtige bzw. mehrkammerige Abdeckhaube oder Abdeckung (z. B. Wärmehalle) zu integrieren, durch welche die Abdicht-, Isolations-, Gasspeicher- und Heizfunktion (z.B. Warmwasserbad oder Warmwassermanschette) wahrgenommen werden kann.

In Ausgestaltung der Erfindung kann eine Verteilvorrichtung innerhalb der Hülle zur rezirkulierenden Verteilung des Perkolats über der Reaktionsmasse vorgesehen sein. Diese kann bevorzugt in der Weise ausgebildet sein, daß ein vorzugsweise mit dem Behälter verbundener, insbesondere in die Boden- oder Wandkonstruktion eines Abroll-Containers integrierter Perkolat-Pufferspeicher über eine Rezirkulationspumpe mit der Verteilvorrichtung verbunden ist.

Die Heizvorrichtung kann gemäß einem weiteren Merkmal der Erfindung als Schlauchheizung ausgebildet sein, die derart mit dem Deckel verbunden ist, daß sie bei sinkendem Füllstand in ständigem Massekontakt verbleibt und somit ein optimaler Wärmeübergang über den gesamten Methanisierungsprozeß gewährleistet ist.

In Ausgestaltung der Erfindung kann der Perkolat-Pufferspeicher über einen Wärmetauscher mit der Heizvorrichtung verbunden sein, so daß die Wärme des anfallenden Perkolats im Perkolat-Pufferspeicher sinnvoll ausgenutzt wird und die rezirkulierende Zuführung dann mit einer geeigneten, meist unter der Anfalltemperatur liegenden Temperatur erfolgen kann.

Neben dem Vorsehen einer Vorrichtung zum dosierten Zuführen von Futterstoffen für die Bakterienstämme hat es sich auch noch als besonders zweckmäßig erwiesen, wenn der Behälter als temporäres Reststofflager und/oder als Transporteinheit und/oder als Verteileinheit ausgebildet ist.

Weitere Vorteile, Merkmale und Einzelheiten der Erfindung ergeben sich aus der nachfolgenden Beschreibung einiger Ausführungsbeispiele sowie anhand der Zeichnung. Dabei zeigen:
- Fig. 1 bis 4: verschiedene Stadien des Verfahrens zur Methanisierung eines Rundballens aus gepreßter Biomasse,
- Fig. 5: eine Vorrichtung, bei der das erfindungsgemäße Verfahren in einem als Fahrsilo ausgebildeten Flachlager mit stückig eingebrachter Biomasse durchgeführt wird.
- Fig. 6: einen Kombi-Einstechdorn mit zusätzlicher Heizeinrichtung,
- Fig. 7: einen Schnitt längs der Linie VII-VII in Fig. 6,
- Fig. 8: einen Schnitt durch einen Flachlager-(Mieten-)Fermenter,
- Fig. 9: einen Schnitt durch einen als Rollcontainer ausgebildeten Fermenter,
- Fig. 10: einen Schnitt durch einen Tauchsack-Fermenter und
- Fig. 11: eine gegenüber Fig. 5 abgewandelte Ausführungsform eines Rollcontainer-Fermenters.

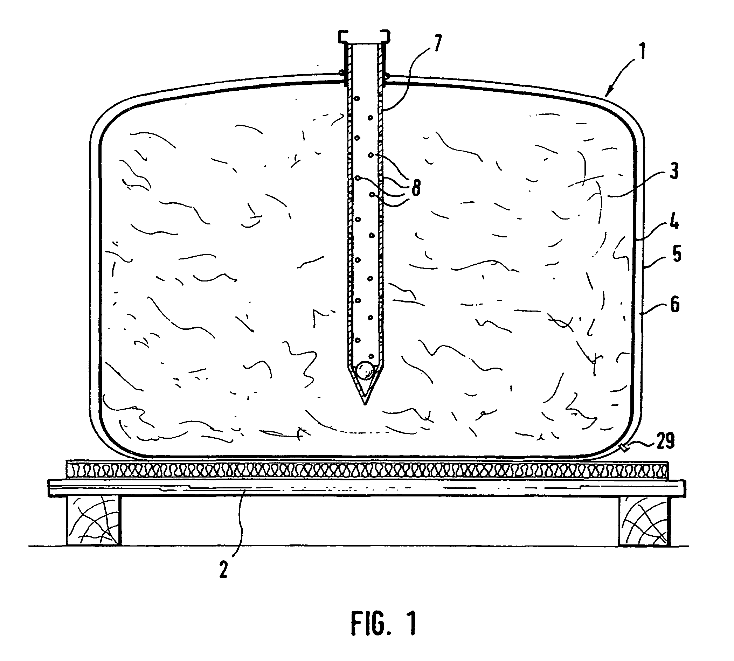

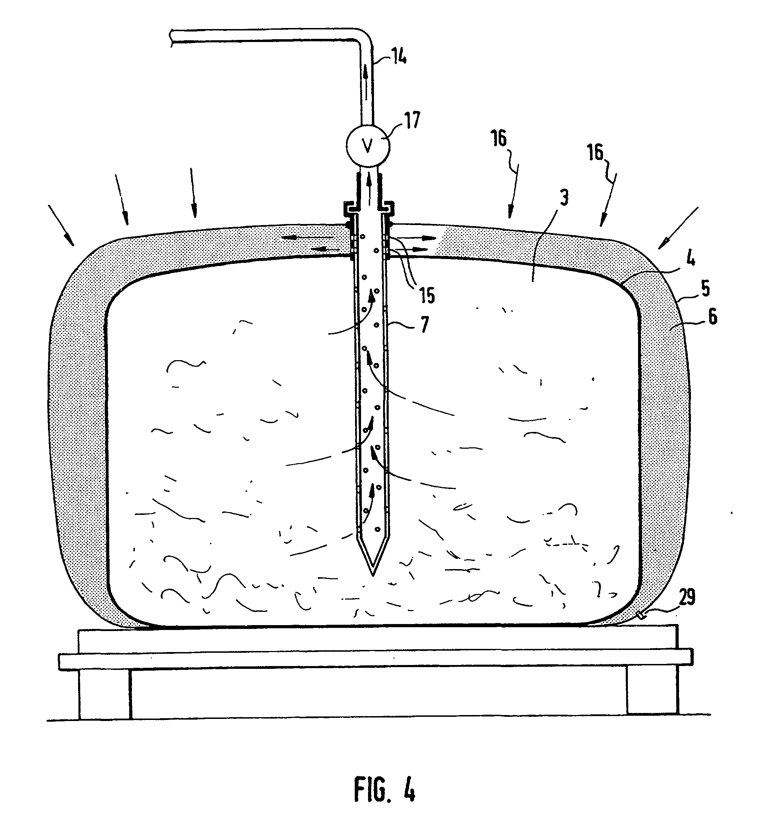

Die Fig. 1 zeigt einen Rundballen 1, der auf einer Palette 2 angeordnet ist, wobei die Biomasse 3 von einer luftdurchlässigen oder luftdichten Transporthülle 4 umgeben sein kann. Die Luftdurchlässigkeit der Transporthülle 4 ist allerdings nicht unbedingt notwendig, wie sich aus der weiteren Beschreibung der Erfindung ergeben wird. Bevorzugt ist um den Rundballen 1 eine zweite luftundurchlässige Hülle 5 angeordnet, innerhalb der sich im Laufe des Verarbeitungsprozesses (vgl. hierzu insbesondere Fig. 4) ein stehendes Gaspolster 6 ausbilden kann, das der Isolierung der Biomasse 3 und damit der Aufrechterhaltung der gewünschten erhöhten Kerntemperatur dient.

In der einfachsten, in den Figuren 1 bis 4 dargestellten Ausführungsform zur Durchführung des erfindungsgemäßen Methanisierungsverfahrens wird in den Rundballen 1 ein Einstechdorn 7 mit einer Vielzahl von Bohrungen 8 eingebracht. Dieser Einstechdorn dient als zentrales Verbindungsorgan für die verschiedenen Ver- und Entsorgungsleitungen.

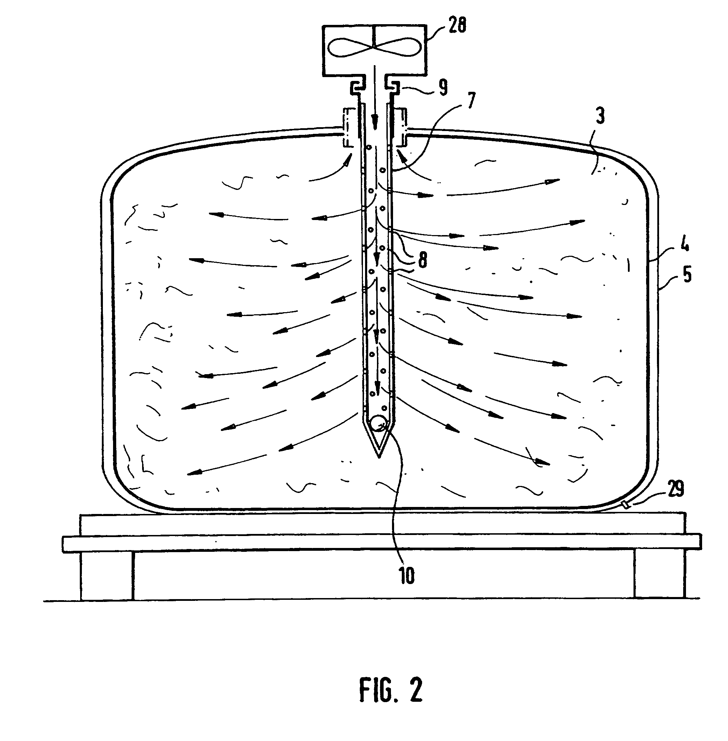

Zunächst wird der Einstechdorn 7 an eine Belüftungsvorrichtung 28, ggf. in Kombination mit einer Befeuchtung, beispielsweise einen Ventilator od. dgl., angeschlossen, so daß Luft ggf., mit Wasser in die Biomasse 3 eingebracht werden kann. Diese Verfahrensstufe ist in Fig. 2 dargestellt. Die Belüftungseinrichtung 28 ist über einen Bajonettflansch 9 an den Einstechdorn 7 angeschlossen, der mit einem Schwimmer 10 versehen ist.

Wenn sich eine ausreichende Erwärmung der Biomasse durch die aerobe Umsetzung eingestellt hat, wird flüssiges oder ausschwemmbares Impfmaterial 11 aus einem an den Einstechdorn 7 angesetzten Behälter 12 ins Innere des Ballens eingebracht und dabei die darin befindliche Luft verdrängt. Wenn das Impfmaterial den Behälter geflutet hat, wird die weitere Zufuhr von Impfmaterial durch das Hochsteigen des Schwimmers 10 und sein Anlegen an die Einfüllöffnung 13 gestoppt.

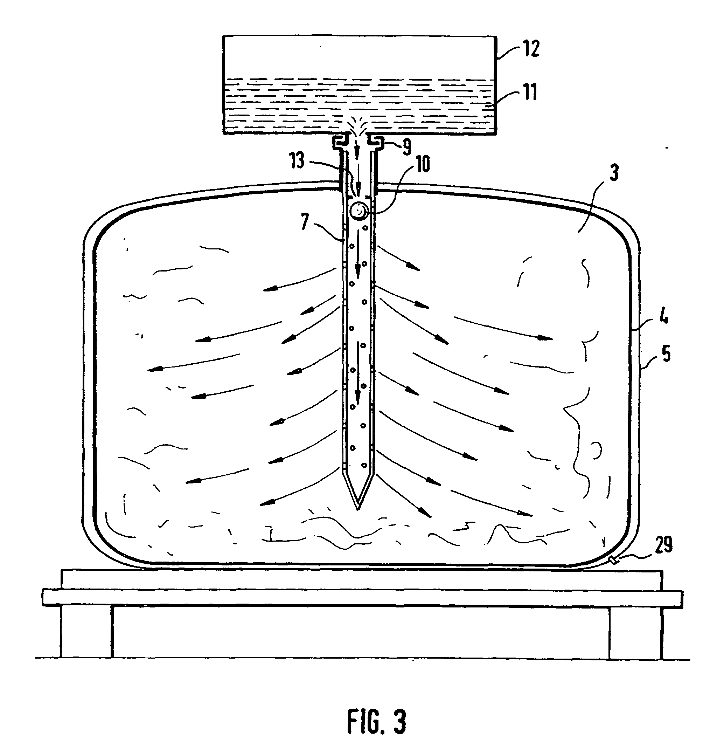

Durch diese Verdrängung von Luft sind die gewünschten anaeroben Bedingungen im Inneren des Ballens hergestellt, so daß nunmehr in der aus der Biomasse und der Impfmasse bestehenden Reaktionsmasse die Methanisierung einsetzt. Der Impfbehälter 12 - der natürlich nur schematisch in Fig. 3 dargestellt ist und nicht unmittelbar jedesmal auf den Ballen aufgesetzt werden muß, sondern auch daneben angeordnet sein kann (Saugheber, kommunizierende Röhren), so daß er über eine Leitung an den Einstechdorn 7 angeschlossen ist - wird abgeklemmt und statt dessen eine Gassammelleitung 14 angeschlossen, wie dies in Fig. 4 gezeigt ist. Das bei der Methanisierung entstehende Gas dringt in den vorher als Impfdorn verwendeten Einstechdorn 7 ein und gelangt über die Öffnungen 15 zwischen die Hülle 4 der Biomasse und die umgebende luftdichte Außenhülle 5. Dabei bildet sich ein großes stehendes Gaspolster 6 aus, durch welches, ggf. in Verbindung mit der durch die Pfeile 16 angedeuteten zusätzlichen Wärmeeinstrahlung durch die Sonne, die notwendige Temperatur zur Methanisierung im Innern der Biomasse 3 gewährleistet wird. Sobald genügend Gas durch die Methanisierung gebildet worden ist, ergibt sich ein ausreichender Überdruck, der das Ventil 17 öffnet, so daß das bei der Methanisierung entstehende Gas kontinuierlich über die Leitung 14 entnommen werden kann.

Verwendet man einen Rundballen, bei dem das bevorzugt feste, schüttfähige Impfmaterial bereits bei der Herstellung des Preßballens mit beigemischt worden ist, so erfolgt statt der Zufügung eines flüssigen Impfmittels in der zweiten Verfahrensstufe gemäß Fig. 3 entweder eine Entlüftung des Ballens oder aber eine Flutung durch Zufügung von vorzugsweise angewärmtem Wasser, durch die einerseits die Luft verdrängt wird und andererseits die notwendige Feuchtigkeit in den meist sehr trockenen Ballen eingebracht wird, die für die Methanisierung erforderlich ist, oder die Luft wird durch das gebildete Biogas verdrängt.

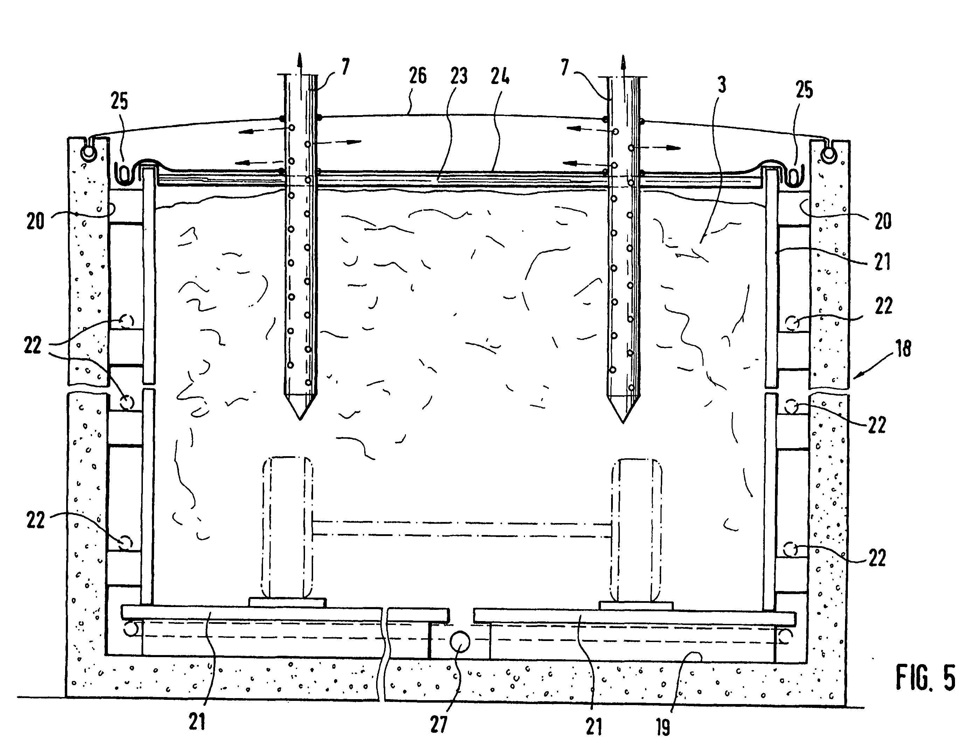

Die Fig. 5 zeigt ein Flachlager zur Durchführung des erfindungsgemäßen Methanisierungsverfahrens, anhand eines Fahrsilos 18. Auf den Boden 19 und an die Wände 20 des Fahrsilos 18 sind einfache Paletten 21 angebracht, die einerseits eine thermische Isolierung bilden und zum anderen die Möglichkeit schaffen, beispielsweise durch Warmwasserleitungen 22 eine zusätzliche Erwärmung der Biomasse 3 zu erzielen. In der ersten Verfahrensstufe wird, angedeutet durch die strichpunktierte Achse und die Räder eines Transportfahrzeugs, die Biomasse 3 eingebracht. Auf diese Biomasse wird ein Laufbrett 23 und darauf eine dehnbare Abdeckfolie 24 aufgebracht, die die Biomasse nach oben dicht überdeckt. Hierzu dienen seitliche Abdichtungen 25. Über der Abdeckfolie 24 ist ggf. nochmals eine weitere Kollektorfolie 26 vorgesehen, die in ihrer Funktion der dichten Außenfolie 5 bei der Rundballenverarbeitung gemäß den Figuren 1 bis 4 entspricht. Bei 27 ist eine Ableitung für Luft, ggf. auch Biogas und Tropfsubstrat und eine Einleitung von Abgas bzw. Biogas angedeutet. Nach dem Befüllen des Fahrsilos und dem Aufbringen der genannten Folien 24 und 26 werden eine Reihe von beabstandeten Einstechdornen 7 eingebracht. Diese können wiederum zu einer Zwangsbelüftung der Biomasse 3 herangezogen werden, obgleich bei der losen Schüttung solcher schüttfähiger Biomassen, wie sie in einem Fahrsilo verarbeitet werden, meist ausreichend Sauerstoff zur Verfügung steht, um die Kernerwärmung, ggf. unterstützt durch Wärmezufuhr über die Warmwasserleitungen 22, zu erzielen. Nach erreichter Kernerwärmung wird wiederum - falls es nicht bereits der Biomasse beigemischt ist - ein fließfähiges Impfmaterial über die Einstechdorne 7 in die Biomasse 3 eingebracht. Bei gut perkolationsfähigem Material kann auch ganz auf technische Verteilhilfen verzichtet werden. Durch das Fluten mit dem lmpfmaterial erfolgt die Luftverdrängung, so daß der Methanisierungsprozeß einsetzen kann. Bei bereits beigemischter Impfmasse muß die Luft entweder abgesaugt und/oder durch Einbringen von die Biomasse ausreichend anfeuchtendem Wasser verdrängt werden, oder die Luftverdrängung erfolgt durch das sich bildende Biogas oder die Einleitung von Abgasen bzw. Biogas.

Wie bereits beim Ausführungsbeispiel nach den Figuren 1 bis 4 beschrieben worden ist, wird in der sich dann anschließenden Methanisierungsstufe das austretende Gas zunächst einen Überdruck zwischen der durch einen Seegerverschluß abgedichteten Abdeckfolie 24 und der Kollektorfolie 26 ausbilden. Dann wiederum öffnet sich ein in Fig. 5 nicht mehr gezeigtes Überdruckventil, das in die Gassammelleitung eingebaut ist, die jeweils an die oberen Enden der Einstechdorne 7 angeschlossen ist. Gegebenenfalls kann die Kollektorfolie 26 auch weggelassen und das Gas unter der Abdeckfolie 24 gesammelt werden.

Entscheidend für beide gezeigte Ausführungsformen ist die Tatsache, daß lediglich eine einmalige Impfung des Biomaterials erfolgt und daß während der Methanisierung keinerlei technologische Prozesse (Homogenisieren, Umpumpen) weder der Reaktionsmasse selbst noch etwaiger Sickersäfte für die Aufrechterhaltung der Methanisierungsstufe erforderlich ist. Dies ergibt eine extrem einfache apparative Ausbildung der zur Durchführung der Methanisierung erforderlichen Vorrichtung, so daß die Methanisierung beispielsweise von in der Landwirtschaft anfallendem Häckselgut direkt auf dem Bauernhof möglich ist. Andererseits läßt das erfindungsgemäße Verfahren es auch zu, die jeweiligen Biomassen, bevorzugt in Form der bereits mehrfach angesprochenen Rund- oder Rechteckballen, oder durch Trocknung, Silierung oder Feuchtstapelung (Festmistlager) bis zu einer gewünschten späteren Methanisierung aufzubewahren und dann diese gezielt entweder einzeln oder in Parallel- oder Serienschaltung auch gemeinsam in einer geeigneten Fermentervariante umzusetzen.

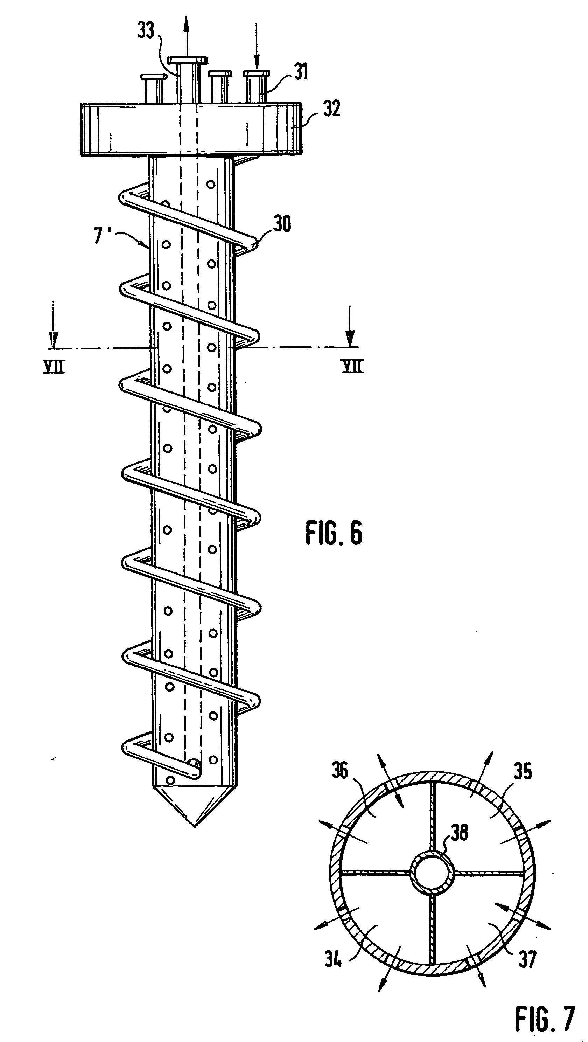

Die Figuren 6 und 7 zeigen einen Kombi-Einstechdorn, mit Hilfe dessen unter anderem auch eine zusätzlich Kernerwärmung der Biomasse bzw. der Reaktionsmasse stattfinden kann. Der Dorn 7' ist von einer Heizwendel 30 umgeben mit einem Vorlaufanschlußstutzen 31 am oberen Flansch 32 und einem Rücklaufstutzen 33 für den im Innern des Kombi-Dorns 7' verlaufenden Rücklauf 38. Die beiden Anschlußstutzen 31 und 33 dienen zum Anschließen an eine äußere Warmwasserquelle, die in beliebiger Weise von einem Solarkollektor oder aber von einer Heizungsanlage, beispielsweise einen Blockheizkraftwerk, das das Biogas verbrennt, stammen kann.

Der Kombidorn 7' ist innen durch Querwände in vier Segmente unterteilt, wobei die Segmente 34 und 35 als Substratzulauf dienen, während die Segmente 36 und 37 wahlweise zur Zuführung von Luft bzw. zum Abführen von Biogas dienen. Selbstverständlich könnte hier auch anstelle von Abgas Biogas eingeblasen werden, um den in der Reaktionsmasse vorhandenen Sauerstoff auszutreiben und somit die Methanisierung des entsprechenden Batch-Ansatzes einzuleiten.

Die Erfindung ist nicht auf die dargestellten Ausführungsbeispiele beschränkt. So wäre es insbesondere auch möglich, die Biomasse in einem Hochsilo umzusetzen oder aber netzgewickelte Rundballen mit einer Zentralspule zu verwenden, wodurch der Einsatz einzelner Einstechdorne 7 überflüssig wird. Diese netzgewickelten Rundballen mit zentralem Spulenkörper eignen sich dabei besonders für eine gleichzeitige Verarbeitung mehrerer Ballen, die zylinderförmig zu einem stabilen Hochsilofermenter gestapelt werden.

Darüber hinaus könnten die Einstechdorne oder die Zentralzylinder so ausgebildet sein, daß sie auch zum Transportieren verwendet werden können (eingeschobener Tragedorn am Frontlader).

Als Flachlager können auch herkömmliche Festmist- oder Silolager dienen und als Substratbehälter ließen sich auch textile Hängesäcke einsetzen (was weiter unten noch an einem speziellen Ausführungsbeispiel gezeigt werden soll).

Neben der Einfachheit der Vorrichtung und des Verfahrens zum Methanisieren von Biomassen gemäß der vorliegenden Erfindung ergibt sich der zusätzliche Vorteil, daß die Nachgärungsphase beliebig lange gemacht werden kann, da nicht wie bei herkömmlichen stationären Anlagen nach einer endlichen Zeit von max. einigen Tagen ein Umschlag des Materials erfolgen muß. Dies wiederum hat zur Folge, daß die Reaktionsmasse völlig vergären kann und somit eine maximale Methanausbeute möglich ist. Nach dem Ausgären kann eine Kompostierung und ein Ausbringen des Restmaterials als feststoffartiger wertvoller organischer Dünger erfolgen, der eine gute Verteilgüte hat und überhaupt keine Umweltbelastung darstellt.

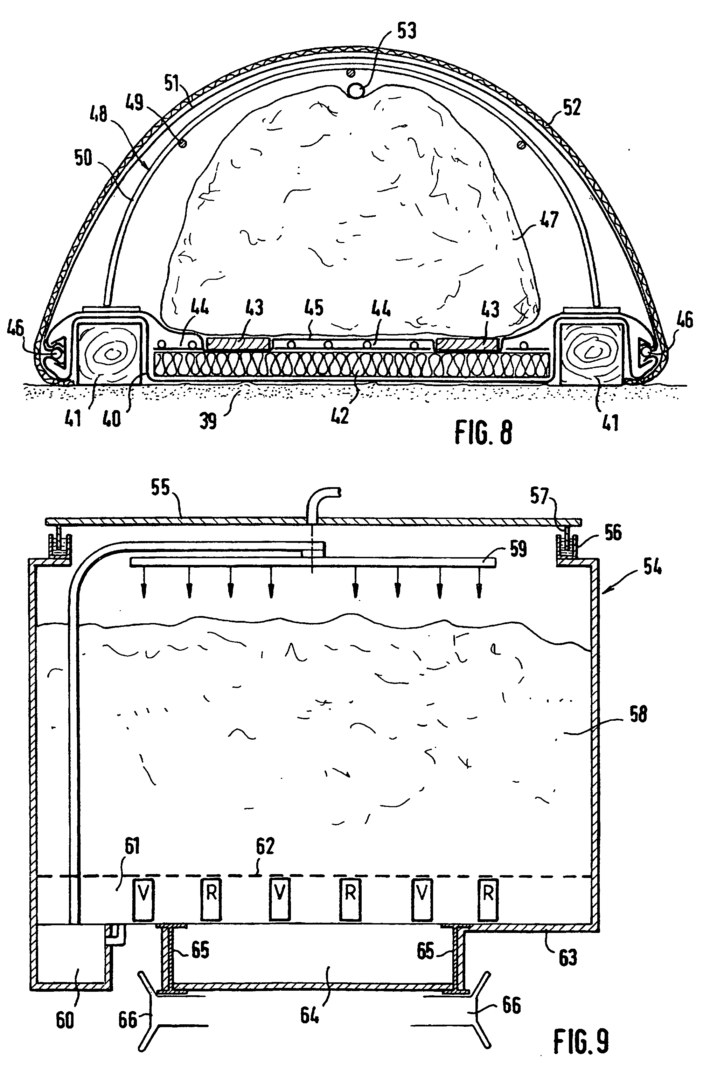

Die Fig. 8 zeigt einen Schnitt durch einen Flachlager-Fermenter, der in sehr einfacher Weise auf einer Sandschüttung 39 aufgebaut sein kann. Über der unteren Abdichtfolie 40, die seitlich über Einfassungsbalken 41 gezogen ist, ist eine Wärmedämmschicht 42 angeordnet, auf der der eigentliche Boden, umfassend Bohlen 43 sowie ein Baustahlgewebe 44 mit eingebauten Heizschlangen angeordnet ist. In der gezeigten Weise ist dabei eine Abdeckfolie 45 über den Boden angeordnet, die gemeinsam mit der unteren Abdichtfolie 40 in seitlichen Seegerverschlüssen 46 gehaltert ist. Über der Biomassenschüttung 47 ist ein Traggestell 48 angeordnet, welches durch Längsträger 49 verbundene gewölbte Bügel 50 umfaßt. Auf diesem ist die obere Abdichtfolie 51 angeordnet, die im gezeigten Ausführungsbeispiel noch von einer Isolier- oder Kollektorfolie 52 überdeckt ist. Gegebenenfalls kann über der Biomassenaufschüttung noch ein Drainrohr 53 angeordnet sein. Halten die Seegerverschlüsse dem anhaltenden Gasdruck nicht stand, kann die Folie 51 auch unter dem Traggestell 48 geführt werden.

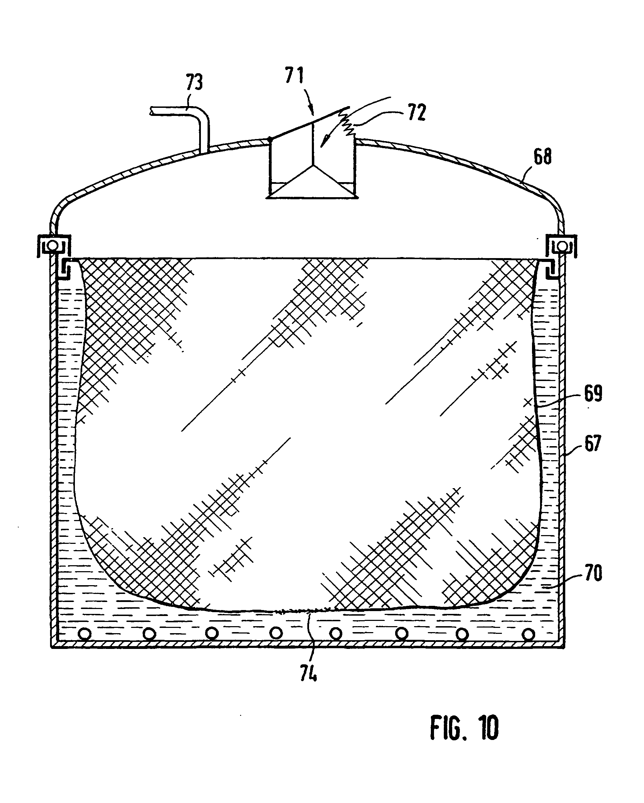

Die Methanisierungsvorrichtung gemäß Fig. 9 ist auf der Basis eines Abroll-Containers 54 aufgebaut, dessen Deckel 55 als Tauchdeckel mit einem in eine wassergefüllte Rinne 56 eintauchenden Rand 57 ausgebildet ist, die als Überdrucksicherung dient. Über dem Substrat 58 ist eine Perkolatverteilvorrichtung 59 angeordnet, die von einer Rezirkulationspumpe 60 mit dem Perkolatsammelraum 61 unterhalb eines perforierten Bodens 62 verbunden ist. Das anfallende Perkolat wird über die Rezirkulationspumpe 60 wieder von oben auf die Biomasse aufgebracht. Innerhalb des Perkolatsammelraums 61 ist eine Tragkonstruktion für den perforierten Boden angeordnet, in die gleichzeitig die Heizung (Wärmetauscher) integriert ist. Dabei bezeichnen die als Träger dienenden Rechteckrohre V jeweils Vorlaufrohre und R Rücklaufrohre dieses Heizsystems.

Neben dem Perkolatsammelraum 61, der direkt über die Pumpe 60 mit der Perkolatverteilvorrichtung 59 verbunden ist, ist in eine Vertiefung des tragenden Bodens 63 des Abroll-Containers 61 noch ein Perkolat-Pufferspeicher 64 integriert, der überschüssig anfallendes Perkolat zumindest vorübergehend aufnimmt, so daß es über eine nicht gezeigte zusätzliche steuerbare Zuleitung zur Rezirkulationspumpe 60 erst dann abgesaugt wird, wenn der Perkolatanfall geringer ist als die wünschenswerterweise rezirkulierend aufgebrachte Menge des Perkolats über die Verteilvorrichtung 59. Bei 65 erkennt man die Traversen für den Tragrahmen des Abroll-Containers und bei 66 modifizierte Führungsrollen am Containerfahrzeug zum Aufziehen und Abrollen des Rollcontainers 54.

Die Fig. 10 zeigt einen Fermenter mit einem Tauchsack. In den beispielsweise aus Kunststoff bestehenden Außenbehälter 67 mit einem abnehmbaren Deckel 68 ist ein wasserdurchlässiger Sack 69, beispielsweise in Form eines Kunststoffnetzes mit enger Maschengröße einhängbar, in welchen sich das zu methanisierende Substrat befindet. Dieser Sack mit dem Substrat hängt in das flüssige Impfmaterial 70, so daß dieses Impfmaterial von allen Seiten nach innen eindiffundieren und die gewünschte Methanisierung bewerkstelligen kann. Nach Art der Gicht in einem Hochofen ist am oberen Ende des Deckels eine Einfüllvorrichtung 71 mit einer Sperrfeder 72 wie Rückschlagklappen angeordnet, so daß auch während des Fermentierens, also während der Biogaserzeugung - bei 73 erkennt man die Biogasabführleitung - ständig weiteres Material von oben nachgefüllt werden kann. Ein derartiger Fermenter eignet sich beispielsweise für die Entsorgung von abgelegenen Gehöften, u. a. von Gebirgshütten. Nach der Methanisierung wird mit Hilfe eines Flaschenzugs od. dgl. der Sack 69 mit dem umgesetzten Biomüll aus dem flüssigen Impfstoff 70, in das er vorher eingetaucht war, herausgehoben. Nach dem Abtropfen kann das vergorene Material einfach als Dünger auf irgendwelche Freiflächen verstreut oder an geeigneter Stelle entgelagert werden. Um dieses Herausnehmen aus dem Sack 69 zu erleichtern, ist am unteren Ende ein Reißleinenverschluß 74 angeordnet, so daß sich der Sack mit einem Zug öffnen läßt und das Material dann - noch am Flaschenzug od. dgl. hängend - nach unten herausrutscht. Der Deckel 68 ist mit dem Behälter 67 in der bereits angesprochenen Weise über einen O-Ringverschluß oder durch Ausbildung des Deckels als Tauchdeckel verbunden.

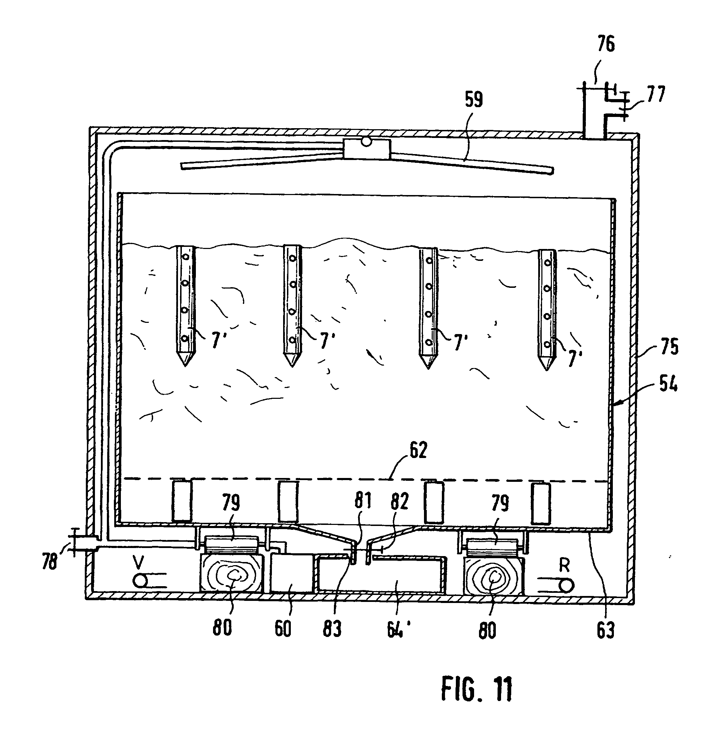

Die Fig. 11 zeigt eine Abwandlung der Methanisierungsvorrichtung gemäß Fig. 9 auf der Basis eines Abroll-Containers 54. Bei dieser Ausführungsform ist der Abroll-Container 54 ein normaler herkömmlicher Abroll-Container ohne Isolierwandungen, wie dies bevorzugt bei der Anordnung nach Fig. 9 der Fall ist. Darüber hinaus ist, abweichend von der Ausgestaltung gemäß Fig. 9, dieser Abroll-Container 54 in einer isolierten gasdichten Hüllkonstruktion 75 angeordnet, also nicht wie bei der Anordnung nach Fig. 9 durch einen Deckel verschlossen. Bei 76 erkennt man den Abgang für das Biogas und bei 77 einen Abgang für die Raumspülung. Mit 59 ist wieder der bereits bekannte Perkolatverteiler angeordnet. Bei 78 erkennt man den Zugang für die Raumspülung. Der temperierbare Perkolatsammler 64' ist in diesem Fall Teil der isolierten gasdichten Hüllkonstruktion, also der Kammer 75. Der Abroll-Container 54 wird auf seinen Rollen 79 über Bohlen 80 in die Hüllkonstruktion 75 gerollt, wodurch der bodenseitige Ablaufstutzen 81 mit einer Verschlußeinrichtung 82 über der Öffnung 83 des Perkolatsammelraums 64' zu liegen kommt. Bei 7' sind bedarfsweise einsetzbar aus den Fig. 1 bis 7 bereits bekannte Einstechdorne zu erkennen, die aber nicht notwendigerweise vorgesehen zu werden brauchen. 60 ist wiederum die Pumpe zum Hochpumpen des Perkolats, so daß es über dem Perkolatverteiler 59 oben auf die Biomasse aufgebracht werden kann. Anstelle des perforierten Einlegebodens könnte auch lediglich eine Sammelmulde in Verbindung mit einer entsprechenden Schrägstellung des Bodens 63 des Abroll-Containers vorgesehen sein. Mit V und R sind der Vor- und der Rücklauf für die Perkolat- und Raumheizung bezeichnet.

## Patentansprüche

1. Verfahren zur Verarbeitung von schüttfähiger, stapelbarer Biomasse (3), umfassend
- eine aerobe Erwärmung der in einem Behälter angeordneten, von einer luftundurchlässigen Hülle (5) umgebenen Biomasse (3),
- eine sich hieran anschließende Methanisierung der inoculierten, in dem Behälter angeordneten und von der luftundurchlässigen Hülle umgebenen Biomasse (3) durch Vergären unter Luftabschluß nach Zusetzen eines Impfmaterials (11) als Einmalzugabe ohne weiteres Nachimpfen,
- Ausbringen des Restmaterials auf Freiflächen, ggfs. nach vorheriger Kompostierung und/oder Zwischenlagerung.

2. Verfahren nach Anspruch 1,
**dadurch gekennzeichnet,**
**daß** als Impfmaterial (11) der Ablauf und/oder das Faulgut einer Biogasanlage verwendet wird.

3. Verfahren nach Anspruch 1 oder Anspruch 2,
**dadurch gekennzeichnet,**
**daß** zur Herstellung der schüttfähigen Biomasse (3) strukturreiche Reststoffe wie Grasschnitt und/oder Strauchschnitt und/oder Stroh und/oder Festmist oder Gülle verwendet werden.

4. Verfahren nach Anspruch 3,
**dadurch gekennzeichnet,**
**daß** die schüttfähige Biomasse mit Impfmaterial in Form von Ablauf aus einer landwirtschaftlichen Biogasanlage oder ausgefaultem Klärschlamm so vermischt werden, daß der Trockenmassegehalt im Gemisch 20 bis 40 Masse-%, vorzugsweise 30 % beträgt und der pH-Wert zwischen 7 und 9, vorzugsweise bei 8,0 liegt.

5. Verfahren nach einem der Ansprüche 1 bis 4,
**dadurch gekennzeichnet,**
**daß** das Verhältnis der Trockenmassen des Impfmaterials (11) und der Biomasse (3) größer als 0,6 ist.

6. Verfahren nach einem der Ansprüche 1 bis 5,
**dadurch gekennzeichnet,**
**daß** die Biomassen (3) organische Reststoffe aus der Landschaftspflege, Lebensmittelindustrie oder Abfälle aus der Biotonne in einer solchen Menge enthält, daß nach Abschluß der Methanisierung noch ein Kompostierungsprozeß in Gang gesetzt werden kann und als Folge die Temperatur des Gemisches auf 40°C bis 60°C, vorzugsweise 50°C, innerhalb von zwei bis fünf Tagen ohne Umsetzen ansteigt.

7. Verfahren nach einem der Ansprüche 1 bis 6,
**dadurch gekennzeichnet,**
**daß** das vergorene Restmaterial nach der Methanisierung als Impfmaterial (11) für einen neuen Batch-Ansatz verwendet wird.

8. Verfahren nach einem der Ansprüche 1 bis 7,
**dadurch gekennzeichnet,**
**daß** das Impfmaterial (11) vor dem Befüllen des Behälters (12), insbesondere bereits bei der Herstellung von Preßballen oder Ladeschwaden mit der Biomasse (3) vermischt wird, wobei die Lagerbedingungen (aerob und unter 20°C) vorzugsweise so gewählt werden, daß eine spontane Methanisierung ausgeschlossen ist.

9. Verfahren nach einem der Ansprüche 1 bis 8,
**dadurch gekennzeichnet,**
**daß** ein fließfähiges Impfmaterial (11) mit einer Biomasse (3) mit hohem Flüssigkeitsbindungsvermögen vermischt wird.

10. Verfahren nach einem der Ansprüche 1 bis 9,
**dadurch gekennzeichnet,**
**daß** das Impfmaterial (11) beim Pressen von Rund- oder Quaderballen zwischen Schichten der Biomasse (3) eingebracht, insbesondere eingesprüht, oder bei Schwadaufnahme in den Schwad oder das Ladegut eingebracht wird.

11. Verfahren nach einem der Ansprüche 1 bis 10,
**dadurch gekennzeichnet,**
**daß** das Impfmaterial (11) in selbstauflösenden "Nestern" in die Biomasse (3) eingebettet ist.

12. Verfahren nach einem der Ansprüche 1 bis 11,
**dadurch gekennzeichnet,**
**daß** ein fließfähiges Impfmaterial (11) verwendet wird, das der Biomasse (3) nach Abschluß der aeroben Kernerwärmung unter Verdrängung der in der Biomasse (3) eingeschlossenen Luft zugeführt wird, daß die Temperatur der Reaktionsmasse einen Wert von ca. 35°C nicht unterschreitet und daß anschließend sofort die anaerobe Vergärungsstufe einsetzt.

13. Verfahren nach einem der Ansprüche 1 bis 12,
**dadurch gekennzeichnet,**
**daß** das Impfmaterial (11) vorgewärmt wird.

14. Verfahren nach einem der Ansprüche 1 bis 13,
**dadurch gekennzeichnet,**
**daß** in der Methanisierungsstufe die Reaktionsmasse nach außen thermisch isoliert wird.

15. Verfahren nach einem der Ansprüche 1 bis 14,
**dadurch gekennzeichnet,**
**daß** in der Methanisierungsstufe eine zusätzliche konventionelle und/oder solare Erwärmung der Reaktionsmasse erfolgt.

16. Verfahren nach einem der Ansprüche 1 bis 15,
**dadurch gekennzeichnet,**
**daß** anfallende Abtropfflüssigkeit (Perkolat) der Reaktionsmasse zur Temperaturhomogenisierung und/oder Beimpfung und/oder zur Regulierung des Trockenmassegehaltes des Substrats (zur Erreichung der Streufähigkeit für Stalldungstreuer) rezirkulierend zugeführt wird.

17. Verfahren nach Anspruch 16,
**dadurch gekennzeichnet,**
**daß** ein Teil des Perkolats in einem Perkolat-Pufferspeicher zwischengespeichert wird.

18. Verfahren nach einem der Ansprüche 1 bis 17,
**dadurch gekennzeichnet,**
**daß** vorzugsweise über eine Begasungseinrichtung oder eine Perkolat-Rezirkulation spezielle Futterstoffe zur Kultivierung von Bakterienstämmen dosiert zugesetzt werden.

19. Verfahren nach einem der Ansprüche 1 bis 18,
**gekennzeichnet durch**
die, vorzugsweise über eine Perkolat-Rezirkulation, dosierte Zufügung von Cofermenten.

20. Verfahren nach einem der Ansprüche 1 bis 19,
**dadurch gekennzeichnet,**
**daß** unterschiedliche Biomassen (3) durch Schichtung, kegelförmige Schüttung, keilförmiges oder röhrenförmiges Einbringen des Impfgutes so "gemischt" werden, daß eine optimale Methanisierung aller Komponenten stattfindet.

21. Vorrichtung zur Durchführung des Verfahrens nach einem der Ansprüche 1 bis 20,
**dadurch gekennzeichnet,**
**daß** die in einer luftundurchlässigen oder luftdurchlässigen Transporthülle verpackte Biomasse (3) von einer als zweite, zumindest zeitweise gasdichte Hülle (5, 26) ausgebildeten Wandung umgeben ist, wobei sich zwischen der Transporthülle und der zweiten Hülle ein stehendes Gaspolster (6) ausbilden kann.

22. Vorrichtung nach Anspruch 21,
**dadurch gekennzeichnet,**
**daß** alle erforderlichen Perkolat-, Impfmaterial- und Gasführungen durch einen oder mehrere zentral oder radial angeordnete Einstechdorn(e) (7) bzw. eine oder mehrere Zentralspule(n) mit oder ohne Flanschverbindungen zugeführt werden.

23. Vorrichtung nach Anspruch 21 oder Anspruch 22,
**dadurch gekennzeichnet,**
**daß** bei formbeständigen Biomassen (3) der Ver- und Entsorgung dienende Bohrungen (8) vorgesehen sind.

24. Vorrichtung nach einem der Ansprüche 21 bis 23,
**dadurch gekennzeichnet,**
**daß** der Ver- und Entsorgung dienende Bahnen durch selbstauflösende Füllkörper gebildet sind.

25. Vorrichtung nach einem der Ansprüche 21 bis 24,
**dadurch gekennzeichnet,**
**daß** der Ver- und Entsorgung dienende Leitungen mittels Schnellverschlüssen miteinander oder mit zentralen Speichern bzw. Verdichtern koppelbar sind.

26. Vorrichtung nach einem der Ansprüche 21 bis 25,
**gekennzeichnet durch**
eingebaute Über- und/oder Unterdruckventile zur Regelung von Gas- bzw. Substratwegen.

27. Vorrichtung nach einem der Ansprüche 21 bis 26,
**gekennzeichnet durch**
einen Kondenswasserablauf (29) in der gasdichten Außenhülle.

28. Vorrichtung nach einem der Ansprüche 21 bis 27,
**gekennzeichnet durch**
eine Verteilvorrichtung innerhalb der Hülle zur rezirkulierenden Verteilung des Perkolats über die Reaktionsmasse.

29. Vorrichtung nach einem der Ansprüche 21 bis 28,
**gekennzeichnet durch**
einen vorzugsweise mit dem Behälter verbundenen, insbesondere in die Boden- oder Wandkonstruktion eines Abroll-Containers integrierten, Perkolat-Pufferspeicher, der über eine Rezirkulationspumpe mit der Verteilvorrichtung verbunden ist.

30. Vorrichtung nach einem der Ansprüche 21 bis 29,
**dadurch gekennzeichnet,**
**daß** ein Perkolat-Pufferspeicher über einen Wärmetauscher mit einer Heizvorrichtung verbunden ist.

31. Vorrichtung nach einem der Ansprüche 21 bis 30,
**dadurch gekennzeichnet,**
**daß** sie eine als Schlauchheizung ausgebildete Heizvorrichtung umfaßt, die derart mit einem Deckel verbunden ist, daß sie bei sinkendem Füllstand in ständigem Massekontakt verbleibt.

32. Vorrichtung nach einem der Ansprüche 21 bis 31,
**dadurch gekennzeichnet,**
**daß** der Behälter als temporäres Lager für das Restmaterial und/oder als Transporteinheit und/oder als Verteileinheit ausgebildet ist.

33. Vorrichtung nach einem der Ansprüche 21 bis 32,
**gekennzeichnet durch**
eine Vorrichtung zum dosierten Zuführen von Futterstoffen für Bakterienstämme.

34. Vorrichtung nach einem der Ansprüche 21 bis 33,
**dadurch gekennzeichnet,**
**daß** die Reaktionsmasse in einem flüssigkeitsdurchlässigen Sack (69) angeordnet ist, der oben offen in einen mit flüssigem Impfmaterial (70) gefüllten Behälter (67)mit einem Deckel (68) getaucht, einhängbar ist.

35. Vorrichtung nach Anspruch 34,
**dadurch gekennzeichnet,**
**daß** der Sack unten mit einer Entleerungsöffnung mit Reisleinenverschluß (74) versehen ist.

36. Vorrichtung nach Anspruch 34 oder Anspruch 35,
**dadurch gekennzeichnet,**
**daß** der Deckel (68) mit einer rückschlaggesicherten Einfüllöffnung nach Art der Gicht eines Hochofens oder Siphons versehen ist.

37. Vorrichtung nach einem der Ansprüche 21 bis 36,
**dadurch gekennzeichnet,**
**daß** mehrere Behälter über spezielle Versorgungsköpfe gekoppelt sind.

38. Vorrichtung nach Anspruch 37,
**dadurch gekennzeichnet,**
**daß** die einzelnen Behälter so gekoppelt sind, daß sie gleichzeitig oder nacheinander in Serien- oder Parallelschaltung betrieben werden können.

## Claims

1. Method for processing a pourable, stackable biomass (3), including
- an aerobic warming of the biomass (3) located within a container and surrounded by an air permeable sleeve (5),
- a subsequent methanising of the inoculated biomass (3) located within the container and surrounded by the air permeable sleeve by means of fermenting under exclusion of air following an addition of an inoculation material (11) as a one-off additive without further inoculation,
- extraction of residual material onto free surfaces, possibly following prior composing and/or interim storage.

2. Method according to Claim 1, **characterised in that** drainage and/or the decomposing matter of a biological gas plant is used as inoculation material (11).

3. Method according to Claim 1 or Claim 2, **characterised in that** structure rich residual materials such as grass cuttings and/or bush cutting and/or straw and/or solid or liquid manure are used for producing the pourable biomass (3).

4. Method according to Claim 3, **characterised in that** the pourable biomass is mixed with inoculation material in the form of drainage from an agricultural biological gas plant or decomposing sludge in such a way that the dry mass content of the mixture is 20 to 40 wt%, preferably 30 wt%, and the pH value 7 to 9, preferably 8.0.

5. Method according to one of the Claims 1 to 4, **characterised in that** the ratio of dry mass of the inoculation material (11) to biomass (3) is greater than 0.6.

6. Method according to one of the Claims 1 to 5, **characterised in that** the biomass (3) includes organic residual material from agricultural processes, the food industry, or biological waste in such quantities that following the completion of the methanising process a composting process can be initiated, and that the temperature of the mixture will rise to between 40°C and 60°C, preferably 50°C, within two to five days without stirring as a consequence.

7. Method according to one of the Claims 1 to 6, **characterised in that** the fermented residual material is used as inoculation material (11) for a new batch following methanising.

8. Method according to one of the Claims 1 to 7, **characterised in that** the inoculation material (11) are mixed with the biomass (3) prior to filling the container (12), especially for producing pressed stacks or loads, whereby the storage conditions (aerobic and below 20°C) are preferably chosen in such a way that a spontaneous methanising is excluded.

9. Method according to one of the Claims 1 to 8, **characterised in that** a flowable inoculation material (11) is mixed with a biomass (3) with a high liquid binding capacity.

10. Method according to one of the Claims 1 to 9, **characterised in that** the inoculation material (11) is inserted between layers of biomass (3) during the pressing of round or square stacks, especially injected, or inserted into the loads during loading into a stack.

11. Method according to one of the Claims 1 to 10, **characterised in that** the inoculation material (11) is embedded into the biomass (3) in the form of self-dissolving "nests".

12. Method according to one of the Claims 1 to 11, **characterised in that** a flowable inoculation material (11) is used which is added to the biomass (3) following completion of the aerobic core warming and displacement of air enclosed within the biomass (3), **in that** the temperature of the reaction mass will not fall below a value of approximately 35°C, and **in that** the anaerobic fermenting step follows immediately.

13. Method according to one of the Claims 1 to 12, **characterised in that** the inoculation material (11) is pre-warmed.

14. Method according to one of the Claims 1 to 13, **characterised in that** the reaction mass is thermally isolated against the outside during the methanising step.

15. Method according to one of the Claims 1 to 14, **characterised in that** an additional conventional and/or solar warming of the reaction mass is carried out during the methanising step.

16. Method according to one of the Claims 1 to 15, **characterised in that** any drainage fluid (percolate) of the reaction mass generated is recirculated for temperature homogenisation and/or inoculation and/or for regulating the dry mass content of the substrate (to achieve pourability for stable dung dispenser).

17. Method according to Claim 16, **characterised in that** a part of the percolate is stored temporarily in a percolate buffer store.

18. Method according to one of the Claims 1 to 17, **characterised in that** special feed stuff for cultivating of bacteria stems is added in doses, preferably by means of a gassing means or percolate recirculation.

19. Method according to one of the Claims 1 to 18, **characterised by** percolate recirculation, preferably by means of adding doses of co-fermenters.

20. Method according to one of the Claims 1 to 19, **characterised in that** different biomasses (3) are mixed through layering, cone-shaped pouring, wedge-shaped or tubular insertion of the inoculation material in such a way that methanisation of all components is optimised.

21. Device for applying the method according to one of the Claims 1 to 20, **characterised in that** the biomass (3) packaged in a non-air permeable or air permeable transport sleeve is surrounded by a sleeve (5, 26) that is gas-tight at least at some time serving as a second wall, whereby an immovable gas buffer (6) can form between the transport sleeve and the second sleeve.

22. Device according to Claim 21, **characterised in that** all necessary percolate, inoculation material, and gas supplies are routed through one or more centrally or radially positioned insertion spike(s) (7), i.e. one or more central coil(s) with or without flange connections.

23. Device according to Claim 21 or Claim 22, **characterised in that** bores (8) serving to supply and drain permanently formed biomasses (3) are envisaged.

24. Device according to one of the Claims 21 to 23, **characterised in that** paths serving supply and drainage are formed through self-dissolving filler shapes.

25. Device according to one of the Claims 21 to 24, **characterised in that** the lines serving for supply and drainage can be coupled with each other or with central stores by means of quick-release connections, i.e. compression fittings.

26. Device according to one of the Claims 21 to 25, **characterised by** fitted over- and underpressure valves for controlling gas, i.e. substrate lines.

27. Device according to one of the Claims 21 to 26, **characterised by** a condensate water drain (29) within the gas-tight outer sleeve.

28. Device according to one of the Claims 21 to 27, **characterised by** a distribution means within the sleeve for recirculating distribution of percolate across the reaction mass.

29. Device according to one of the Claims 21 to 28, **characterised by** a percolate store preferably connected with a container, especially integrated into the floor or wall construction of a rollout container, and connected with the distribution means via a recirculation pump.

30. Device according to one of the Claims 21 to 29, **characterised in that** a percolate buffer store is connected with heating means via a heat exchanger.

31. Device according to one of the Claims 21 to 30, **characterised in that** the same incorporates a heating means in the form of a pipe heater which is connected with a lid in such a way that the same remains in constant contact with mass when the fill level falls.

32. Device according to one of the Claims 21 to 31, **characterised in that** the container takes the form of a temporary store for the residual material and/or as a transport unit and/or as a distribution unit.

33. Device according to one of the Claims 21 to 32, **characterised by** a means for adding doses of feed stuff for bacteria stems.

34. Device according to one of the Claims 21 to 33, **characterised in that** the reaction mass is positioned within a liquid permeable sack (69) open at the top, which can be hung and submersed into a container (67) with a lid (68) filled with liquid inoculation material (70).

35. Device according to Claim 34, **characterised in that** the sack is equipped with a drainage opening with a rip-cord closure (74) at the bottom.

36. Device according to Claim 34 or Claim 35, **characterised in that** the lid (68) is equipped with a non-return filler opening of the type used for furnaces or siphons.

37. Device according to one of the Claims 21 to 36, **characterised in that** several containers are coupled via special supply headers.

38. Device according to Claim 37, **characterised in that** the individual containers are coupled in such a way that the same can be operated simultaneously or subsequently in series or in parallel.

## Revendications

1. Procédé de traitement de biomasse (3) en vrac gerbable, comprenant
- le chauffage aérobie de la biomasse (3) disposée dans une cuve et entourée d'une enveloppe (5) imperméable à l'air,
- une méthanisation subséquente de la biomasse (3) ayant fait l'objet de l'inoculation, disposée dans la cuve et entourée de l'enveloppe imperméable à l'air, par fermentation à l'abri de l'air, après addition d'un matériau d'inoculation (11) en tant qu'ajout unique, sans inoculation ultérieure,
- épandage de la matière résiduelle sur des surfaces libres, le cas échéant après compostage préalable et/ou stockage intermédiaire.

2. Procédé selon la revendication 1, **caractérisé en ce que** le matériau d'inoculation (11) utilisé est constitué de l'égout et/ou du produit putréfié d'une installation de biogaz.

3. Procédé selon la revendication 1 ou la revendication 2, **caractérisé en ce que** des matières résiduelles à structure marquée, telles que de l'herbe coupée et/ou des arbustes coupés et/ou de la paille et/ou du fumier solide ou du purin, sont utilisées pour la fabrication de la biomasse (3) en vrac.

4. Procédé selon la revendication 3, **caractérisé en ce que** la biomasse en vrac est mélangée avec un matériau d'inoculation constitué des effluents d'une installation de biogaz agricole ou de boues de curage putrides, de manière telle que la teneur en matière sèche du mélange soit comprise entre 20 et 40 pour cent de masse et soit de préférence de 30 %, et que le pH soit compris entre 7 et 9 et soit de préférence de 8,0.

5. Procédé selon une des revendications 1 à 4, **caractérisé en ce que** le rapport des matières sèches du matériau d'inoculation (11) et de la biomasse (3) est supérieur à 0,6.

6. Procédé selon une des revendications 1 à 5, **caractérisé en ce que** la biomasse (3) contient des matières résiduelles organiques provenant de la préservation du paysage, de l'industrie alimentaire ou de déchets ménagers biologiques, et cela en une quantité telle qu'à la fin de la méthanisation, il existe la possibilité de déclencher un processus de compostage et que cela ait pour conséquence une élévation de la température du mélange jusqu'à 40 à 60 °C, de préférence à 50 °C, en l'espace de deux à cinq jours, sans conversion.

7. Procédé selon une des revendications 1 à 6, **caractérisé en ce que**, après la méthanisation, les matières résiduelles fermentées sont utilisées comme matériau d'inoculation (11) pour la préparation d'un nouveau lot.

8. Procédé selon une des revendications 1 à 7, **caractérisé en ce que** le matériau d'inoculation (11) est mélangé avec la biomasse (3) avant le remplissage de la cuve (12), notamment déjà lors de la formation de balles comprimées ou d'andains à charger, les conditions de stockage (aérobies et inférieures à 20°C) étant choisies de manière à exclure une méthanisation spontanée.

9. Procédé selon une des revendications 1 à 8, **caractérisé en ce qu'**un matériau d'inoculation (11) fluide est mélangé avec une biomasse (3) présentant un grand pouvoir de liaison de liquide.

10. Procédé selon une des revendications 1 à 9, **caractérisé en ce que** le matériau d'inoculation (11) est introduit, notamment par pulvérisation, entre des couches de la biomasse (3), lors de la compression de balles rondes ou parallélépipédiques, ou est introduit dans les andains lors de leur ramassage ou dans les matières à charger.

11. Procédé selon une des revendications 1 à 10, **caractérisé en ce que** le matériau d'inoculation (11) est placé dans la biomasse (3), dans des "nids" à auto-dissolution.

12. Procédé selon une des revendications 1 à 11, **caractérisé en ce que** l'on utilise un matériau d'inoculation (11) fluide qui est ajouté à la biomasse (3) à la fin du chauffage au coeur aérobie, en chassant l'air inclus dans la biomasse (3), **en ce que** la température de la masse de réaction ne passe pas en dessous d'environ 35°C et **en ce que** la phase de fermentation anaérobie commence immédiatement après.

13. Procédé selon une des revendications 1 à 12, **caractérisé en ce que** le matériau d'inoculation (11) est préchauffé.

14. Procédé selon une des revendications 1 à 13, **caractérisé en ce que** pendant la phase de méthanisation, la masse de réaction est isolée thermiquement vis-à-vis de l'extérieur.

15. Procédé selon une des revendications 1 à 14, **caractérisé en ce que** la phase de méthanisation comporte un chauffage supplémentaire classique et/ou solaire de la masse de réaction.

16. Procédé selon une des revendications 1 à 15, **caractérisé en ce que** le liquide d'égouttage (de percolation) produit est amené par recirculation à la masse de réaction à des fins d'homogénéisation de la température et/ou d'inoculation et/ou de régulation de la teneur en matière sèche du substrat (pour obtenir l'aptitude à l'épandage pour des épandeurs de fumier).

17. Procédé selon la revendication 16, **caractérisé en ce qu'**une partie du produit de percolation fait l'objet d'un stockage intermédiaire dans un réservoir tampon pour produit de percolation.

18. Procédé selon une des revendications 1 à 17, **caractérisé en ce que** des substances nutritives spéciales sont ajoutées de façon dosée, de préférence via un dispositif d'injection de gaz ou un moyen de recirculation de produit de percolation, en vue de cultiver des souches bactériennes.

19. Procédé selon une des revendications 1 à 18, **caractérisé en ce qu'**il comprend l'addition dosée de coferments, de préférence par l'intermédiaire d'une recirculation de produit de percolation.

20. Procédé selon une des revendications 1 à 19, **caractérisé en ce que** des biomasses (3) différentes sont "mélangées" par disposition par couches, par disposition en vrac en forme de cône ou par introduction cunéiforme ou tubulaire du matériau d'inoculation, de manière à obtenir une méthanisation optimale de tous les composants.

21. Dispositif pour la mise en oeuvre du procédé selon une des revendications 1 à 20, **caractérisé en ce que** la biomasse (3), qui est emballée dans une enveloppe de transport imperméable à l'air ou perméable à l'air, est entourée d'une paroi réalisée comme seconde enveloppe (5, 26) au moins temporairement étanche aux gaz, un coussin de gaz (6) stationnaire pouvant se former entre l'enveloppe de transport et la seconde enveloppe.

22. Dispositif selon la revendication 21, **caractérisé en ce que** toutes les alimentations nécessaires en produit de percolation, en matériau d'inoculation et en gaz sont assurées par une ou plusieurs tige(s) à pointe (7), disposée(s) de manière centrale ou radiale, ou une ou plusieurs bobine(s) centrale(s), avec ou sans raccords à brides.

23. Dispositif selon la revendication 21 ou la revendication 22, **caractérisé en ce que** pour des biomasses (3) de forme stable, il est prévu des orifices (8) servant à l'alimentation et à l'évacuation.

24. Dispositif selon une des revendications 21 à 23, **caractérisé en ce que** des voies servant à l'alimentation et à l'évacuation sont formées par des corps de remplissage à auto-dissolution.

25. Dispositif selon une des revendications 21 à 24, **caractérisé en ce que** des conduites, servant à l'alimentation et à l'évacuation, peuvent être couplées entre elles ou avec des réservoirs ou des compresseurs centraux à l'aide de raccords rapides.

26. Dispositif selon une des revendications 21 à 25, **caractérisé en ce qu'**il comprend des soupapes de surpression et/ou de dépression intégrées pour réguler les trajets des gaz et des substrats.

27. Dispositif selon une des revendications 21 à 26, **caractérisé en ce qu'**il comporte un orifice d'écoulement (29) d'eau de condensation qui est aménagé dans l'enveloppe extérieure étanche aux gaz.

28. Dispositif selon une des revendications 21 à 27, **caractérisé en ce qu'**il comprend un moyen de répartition à l'intérieur de l'enveloppe, en vue de répartir par recirculation le produit de percolation sur la masse de réaction.

29. Dispositif selon une des revendications 21 à 28, **caractérisé en ce qu'**il comprend un réservoir tampon de produit de percolation qui est de préférence lié à la cuve, est notamment intégré dans la construction de plancher ou de paroi d'une cuve mobile et est relié au dispositif de répartition par l'intermédiaire d'une pompe de recirculation.

30. Dispositif selon une des revendications 21 à 29, **caractérisé en ce qu'**un réservoir tampon de produit de percolation est relié à un dispositif de chauffage par l'intermédiaire d'un échangeur de chaleur.

31. Dispositif selon une des revendications 21 à 30, **caractérisé en ce qu'**il comprend un dispositif de chauffage réalisé sous forme de chauffage à tuyaux souples, qui est relié à un couvercle de manière telle qu'il reste en contact permanent avec la masse, même lorsque le niveau baisse.

32. Dispositif selon une des revendications 21 à 31, **caractérisé en ce que** la cuve est réalisée comme moyen de stockage temporaire pour les matières résiduelles et/ou comme unité de transport et/ou comme unité de distribution.

33. Dispositif selon une des revendications 21 à 32, **caractérisé en ce qu'**il comprend un moyen d'alimentation dosée de substances nutritives pour des souches bactériennes.

34. Dispositif selon une des revendications 21 à 33, **caractérisé en ce que** la masse de réaction est placée dans un sac (69) perméable aux liquides, dont l'extrémité supérieure est ouverte et qui peut être suspendu dans une cuve (67) remplie d'un matériau d'inoculation (70) liquide et équipée d'un couvercle (68).

35. Dispositif selon la revendication 34, **caractérisé en ce que** le sac comporte à son extrémité inférieure un orifice d'évacuation muni d'une fermeture à corde de déchirure (74).

36. Dispositif selon la revendication 34 ou la revendication 35, **caractérisé en ce que** le couvercle (68) est pourvu d'une ouverture de remplissage à protection anti-retour, à la manière d'un gueulard de haut fourneau ou d'un siphon.

37. Dispositif selon une des revendications 21 à 36, **caractérisé en ce que** plusieurs cuves sont couplées par l'intermédiaire de têtes d'alimentation spéciales

38. Dispositif selon la revendication 37, **caractérisé en ce que** les différentes cuves sont couplées de manière telle qu'elles puissent être exploitées simultanément ou successivement, dans un branchement en série ou en parallèle.
